# EUROPEAN PATENT SPECIFICATION

(11) **EP 1 049 718 B1**
(45) Date of publication and mention of the grant of the patent: **05.04.2006**
(21) Application number: 99907361.2
(22) Date of filing: 11.01.1999
(51) Int. Cl.: C07K 16/28, A61K 39/395, C12N 5/20, C12P 21/08

(54) **USE OF THE ANTIBODY 271.14D9.F8 (DSM ACC2331) TO INHIBIT IN VITRO ALPHAVBETA6-INTEGRIN ATTACHMENT TO FIBRONECTIN**
VERWENDUNG DES ANTIKÖRPERS 271.14D9.F8 (DSM ACC2331) UM DIE BINDUNG ZWISCHEN ALPHAVBETA6-INTEGRIN UND FIBRONECTIN IN VITRO ZU HEMMEN
UTILISATION DE L'ANTICORPS 271.14D9.F8 (DSM ACC2331) POUR INHIBER IN VITRO LA FIXATION DE L'INTEGRINE ALPHAVBETA6 A LA FIBRONECTINE

(30) Priority: 23.01.1998 EP 98101108
(43) Date of publication of application: 08.11.2000
(73) Proprietor: Merck Patent GmbH, 64293 Darmstadt (DE)
(72) Inventor: GOODMAN, Simon, D-64286 Darmstadt (DE); DIEFENBACH, Beate, D-64289 Darmstadt (DE); MITJANS, Francesc, E-08700 Igualada (ES); CARCELLER, Ana, E-08028 Barcelona (ES); ROSELL-VIVES, Elisabet, E-08008 Barcelona (ES)
(86) International application number: PCT/EP1999/000101
(87) International publication number: WO 1999/037683

(56) References cited:
- EP-A- 0 719 859
- MITJANS F. ET AL.: "An anti-alpha v-integrin antibody that blocks integrin function inhibits the development of a human melanoma in nude mice." JOURNAL OF CELL SCIENCE, vol. 108, no. 8, August 1995, pages 2825-2838, XP000566392
- LEHMANN M. ET AL.: "A monoclonal antibody inhibits adhesion to fibronectin and vitronectin of a colon carcinoma cell line and recognizes the integrins alphavbeta (3, 5 and 6)." CANCER RESEARCH, vol. 54, 15 April 1994, pages 2102-2107, XP002103619
- KEMPERMAN H. ET AL.: "alphaV Integrins on HT-29 colon carcinoma cells: adhesion to fibronectin is mediated solely by small amounts of alphaVbeta6, and alphaVbeta5 is codistributed with actin fibers." EXPERIMENTAL CELL RESEARCH, vol. 234, no. 1, 10 July 1997, pages 156-64, XP002103620
- BREUSS J.M. ET AL.: "Expression of the beta 6 integrin subunit in development, neoplasia and tissue repair suggests a role in epithelial remodeling." JOURNAL OF CELL SCIENCE, vol. 108, no. 6, June 1995, pages 2241-51, XP002103621

## Description

### TECHNICAL FIELD OF THE INVENTION

The invention relates to the use of the monoclonal antibody deriving from the hybridoma cell line, having the designation 271.14D9.F8, deposited under accession number DSM ACC2331, for selectively inhibiting *in vitro* the attachment of an αvβ6 integrin bearing cell to the integrin substrate fibronectin.

### BACKGROUND OF THE INVENTION

Integrins are a super-family of cell surface adhesion receptors which control the attachment of cells with the solid extracellular environment - both to the extracellular matrix (ECM), and to other cells. Adhesion is of fundamental importance to a cell; it provides anchorage, cues for migration, and signals for growth and differentiation. Integrins are directly involved in numerous normal and pathological conditions, and as such are primary targets for therapeutic intervention. Integrins are integral transmembrane proteins, heterodimers, whose binding specificity depends on which of some 15 α-chains is combined with which of some 8 β-chains. The integrins are classified in four overlapping subfamilies, containing the β1, β2, β3 or αv chains, and a particular cell may express several different integrins from each subfamily. The last decade has shown that integrins are major receptors involved in cell adhesion. Reports concerning integrins are given, for example, by E. Ruoslahti (J. Clin. Invest., 1991, 87) and R. O. Hynes (Cell, 1992, 69), and so may be a suitable target for therapeutic intervention.

Excepting erythrocytes, all human cells express one or more integrins. Their functions are regulated at many levels, but primarily, their ligand specificity depends on which α-chain associates with which β- chain in the heterodimer and on the activation state of the integrins (Hynes, 1992; Diamond and Springer, 1994). The cellular background in which the integrins operate (Chan and Hemler, 1993), and the splice-variant form of the integrin which is used (Delwel et al., 1993) may also affect specificity. Given these complexities, one of

The history of integrin research has shown that reagents that can specifically block integrin function are decisive factors in functional analysis, from the function blocking CSAT-antibody, which first defined an integrin β1-chain (Neff et al., 1982), to the numerous vital later examples (eg. P1D6. P1B5(Wayner and Carter, 1987), P4C10 (Carter et al., 1990), and LM609 (Cheresh and Spiro, 1987)): the field is absolutely dependent on such reagents.

The αv-series integrins are now seen to be a major subfamily, with both classical, and novel functions. As well as classically mediating cell attachment and spreading (Pytela et al., 1985; Cheresh, 1991), α v integrins have also been implicated in cell locomotion (Seftor et al., 1992), in receptor internalization (Panetti and McKeown Longo, 1993a; Panetti and McKeown Longo, 1993b), as virus co-receptors (Wickham et al., 1993), in management of the extracellular protease cascades (de Boer et al., 1993), and as regulators of tumor progression (Felding-Habermann et al., 1992). The specificities of the five known αv-series integrins, αvβ1 (Zhang et al., 1993), -β3 (Pytela et al., 1985, Cheresh et al., 1987), -β5 (Cheresh et al., 1989), -β6 (Busk et al., 1992) and -β8 (Moyle et al., 1991), have been partly defined, and they seem to exclusively recognize ligands bearing the RGD- (NH2-arginine-glycine-aspartic acid- COOH) tripeptide sequences, including those in vitronectin (αvβ1, αvβ3, αvβ5), fibronectin (αvβ1, αvβ3, αvβ5, αvβ6), and von Willebrand factor, fibrinogen, and osteopontin (αvβ3) (e.g. 1991; Busk et al., 1992; Zhang et al., 1993; Denhardt and Guo, 1993; Smith and Cheresh, 1990;).

It is known that αvβ3 and αvβ5 are involved in tumor angiogenesis, while αvβ6 is a co-stimulator of colon carcinoma cell proliferation and is up-regulated during inflammatory epithelial proliferation and epithelia carcinogenesis (Breuss et al., J. Cell. Sci. 108:2241-2251, 1995). Its expression seems to mark neo-hyperproliferative epithelia, (Zambruno et al. J. Cell. Biol. 129, 853-865 (1995)). Integrin over-expression can produce a psoriasis-like model condition. Specific antagonists and agonists of αvβ6-integrins may thus have wide therapeutic potential.
RGD-peptides block αvβ6 binding to fibronectin, but they also block other cell surface fibronectin receptors, including α5β1 (Pytela, R, et al. Cell (1985) 40, 191-198) and αvβ3 (Pytela, R.Proc. Natl. Acad. Sci. USA 82, 5766-70 (1985)). Inhibitory antibodies have been described that target all αv integrins (Lehmann et al., Cancer Res. 54, 2102-2107 (1994)) and the αvβ3 or αvβ5 complexes (Weinacker et al., J. Biol.Chem, 269, 6940-6948 (1994)), but no reagent is known to selectively inhibit αvβ6.
It has been previously described a series of antibodies that bind human integrin αv-chains including the "non-inhibitory" Mab 14D9.F8 (Mitjans et al., J. Cell. Sci. **108**, 2825.2838 (1995)).

One of the antibodies disclosed by Mitjans et al, is 17E6, which antibody binds to human integrin αv and inhibits all integrins containing this chain. As such it is "unselective", Further, Mitjans et al. disclose the binding properties of 14D9,F8 which is shown to inhibit weakly the interaction UCLA-P3 (expressing αvβ3) - vitronectin. However, Mitjans et al. do not disclose the surprisingly high selectivity of 14D9.F8 in that it blocks the interactions of a defined subclass of av-integrins (αvβ6, αvβ3) with their corresponding substrates, and the same time shows no reactivity on αvβ5,

Lehman et al. (Cancer Res. 54, 2102-2107 (1994)) described the monoclonal antibody (69-6-5) that - similar to 17E6 of Mitjans et al. - binds to the integrin chain αv and inhibits all integrins containing this chain. As such it is "unselective" for αv-series integrins.

Kemperman et al. (Exp. Cell Res. 234, 154-164 (1997)) disclose the antibody L230, which - like 17E6 and 69-6-5 - binds the αv chain, and inhibits all αv-series integrins, and the antibody 33B6 which binds the β6 chain, but is non inhibitory. L230 is similar to 17E6 of Mitjans et al., 33B6 is not inhibitory and has not been described as affecting integrin αvβ6 / ligand interaction.

In EP 0 719 859 A1, the antibody of Mitjans et al. - 17E6 - is used that binds and inhibits all αv integrins.

Breuss et al, (J, Cell Sci., 108, 2241-2251 (1995)) disclose the monoclonal antibodies 9G6 and R6G9 that bind to but do not inhibit the integrin b6 chain. These antibodies are not inhibitory and have not been described as affecting integrin αvβ6 / ligand interaction.

As can be seen from the literature discussed above, several integrin binding antibodies are known in the art. However, these antibodies including 14D9,F8, mentioned by Mitjans et al., do not distinguish one av integrin from another, or are at least not known or even expected to have this important property.

Hence, the need exists to possess an antibody that blocks the interactions of a defined subclass of αv-integrins (αvβ6, αvβ3) with their corresponding substrates, and the same time shows no reactivity on αvβ5.

Suprisingly, it was found by the inventors of the instant patent application that antibody 14D9.F8 can be used for selectively inhibiting *in vitro* the attachment of an αvβ6 integrin bearing cell to the integrin substrate fibronectin.

### GENERAL, MATERIALS, FIGURES, TABLES

Purification and sources of proteins, cell lines and their culture, ELISA, adhesion assays, FACS analysis and the production and characterisation of the antibodies used have been described in detail elsewhere (Mitjans, F. et al, J. Cell Sci. 108, 2825-2838 (1995)). The Mabs 14D9.F8, 11D1 and 17E6 were purified from serum-free hybridoma supernatants by protein-A affinity chromatography followed by removal of endotoxins and were > 99% pure by SDS-PAGE (Mitjans, F. et al. J. Cell. Sci. 108, 2825-2838(1995)). Mouse monoclonal 11D1 is an IgG₁ is directed against integrin β5. Molecular mass of 155 KDa is assumed for murine IgG. The preparation of transmembrane truncated recombinant αvβ6 was essentially as described in Weinacker, A. et al. J. Biol. Chem. 269, 6940-48 (1994). P3D10 (Agrez, M. et al. J. Cell. Biol. 127, 547-556 (1994)), E. Wayner (University of Minnesota, Minneapolis), and 4B7, J. Marshall (IRCF, London) were generous gifts. The antibody selectivity of the antibodies used is summarized in Tab. 1 and the integrin profile of the cell lines used is summarized in Tab. 2. The cell line was deposited under accession number DSM ACC2331 at the Deutsche Sammlung für Mikroorganismen, Braunschweig, FRG.

The DNA- and amino acid sequences include also slightly varied or altered sequences such as mutants and variants which can be obtained intentionally or randomly by chemical or physical processes. Generally, all such mutants and variants are included which show the described properties and functions.

The term antibody includes also, as a rule, antibody fragments such as Fab', F(ab')₂ or single-chain Fvs. These fragments can be produced by usual standard techniques

### Table 1

The derivation and characterisation of these antibodies is defined in the cited literature, and in this description.

### Table 2

The integrin expression profiles of these lines has been defined by surface labelling and FACS anaylsis or immunoprecipitation.(FACS= Fluorescence Activated Cell Sorter)

### Figure 1

### Mab 14D9. F8 binds αv-integrin chains in several contexts:

Recombinant soluble αvβ3 (A), placental αvβ5 (B), recombinant soluble αvβ6 (C), and platelet αllbβ3 (D) (all at 1 µg ml⁻¹) were absorbed to 96-well ELISA plates and probed with the indicated concentrations of Mabs 14D9.F8 (*solid squares*), 17E6 (*solid circles*), 4B7 (*solid triangles up*), 11D1 (*solid triangles down),* AP3 (*solid diamonds*), E7P6 (*open circles*) was used as hybridoma supernatant, the 5 µg/ml point representing a supernatant dilution of 1:5. Bound antibody was detected with peroxidase-conjugated anti-mouse IgG. Error Bars = SD (n=3)

### Figure 2

### Mab 14D9.F8 inhibits cell adhesion to fibronectin but not to vitronectin:

HT-29 colon carcinoma (A,B), UCLA-P3 lung carcinoma (C,D), WM-164 melanoma (E,F) were allowed to attach to plates coated form 5 µg ml⁻¹ solutions of fibronectin (A,C,E) or vitronectin (B,D,F) in the presence of the indicated concentrations of Mabs 14D9.F8 (*solid squares*), 17E6 (*solid circles*), P1F6 (*solid triangles down*), P4C10 (*solid triangles up*), LM 609 (*solid diamond*). After 1 h unbound cells were washed away, and attached cells detected by estimating lysozomal hexosaminidase. Attachment has been normalized using maximum cells attached as 100%. Actual cell attachments on fibronectin and vitronectin respectively were HT-29 (27%, 53%), UCLA-P3 (42%, 64%), WM 164 (84%, 50%). Error Bars = SD (n=3)

### Figure 3

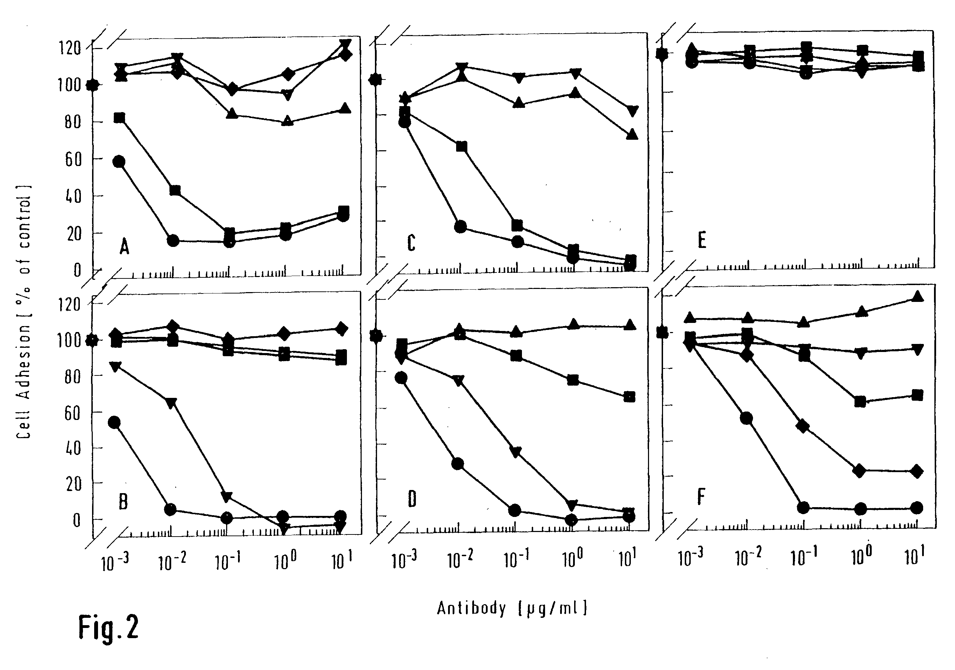

*Mab 14D9.F8 selectively acts on the* αvβ6 *integrin.*
SW480 cells mock transfected (mock) or transfected with full-length human β6 integrin (Beta-6), were allowed to attach to plates coated with fibronectin (FN) or vitronectin (VN) for 1 h in the presence of 10 µg ml⁻¹ antibodies as described in Fig.2. Error Bars = SD (n=3)

### References

1. Weinacker,A., Chen, A., Agrez, M., Cone, R. I. Nishimura,S., Wayner, E., Pytela, R., and Sheppard, D. (1994) J. Biol. Chem. 269, 6940-6948
2. Agrez, M., Chen, A., Cone, R. I., Pytela, R., and Sheppard, D. (1994) J. Cell Biol. 127, 547-556
3. Mitjans, F., Sander, D., Adan, J., Sutter,A., Marinez, J. M. , Jäggle, C., Moyano, J., Kreysch, H.-G., Piulats, J., and Goodman, S.L. (1995) J. Cell Sci. 108, 2825-2838
4. Cheresh, D. A. and Spiro, R.C. (1987) J. Biol. Chem. 262, 17703-17711
5. Houghton, A. N., Eisinger, M. Albino, A. P., Caimcross, J.G., and Old, L.J. (1982) J. EXP. MED. 156, 1755-1766
6. Carter, W., Wayner, E., Bouchard, T., and Kaur, P. (1990) J. Cell Sci. 110, 1387-1404
7. Furihata, K., Nugent, D. J., Bissonette, A., Aster, R. H., and Kunicki, T. J. (1987) J. Clin. Invest. 80, 1624-1630
8. Ebert, E. C. (1996) Dig. Dis. Sci. 41, 1551-1556
9. Koretz, K. Bruderlein, S. Henne, C., Fietz, T., Laque, M., and Moller, P. (1994) Virchows Arch. 425, 229-236

### DETAILED DESCRIPTION

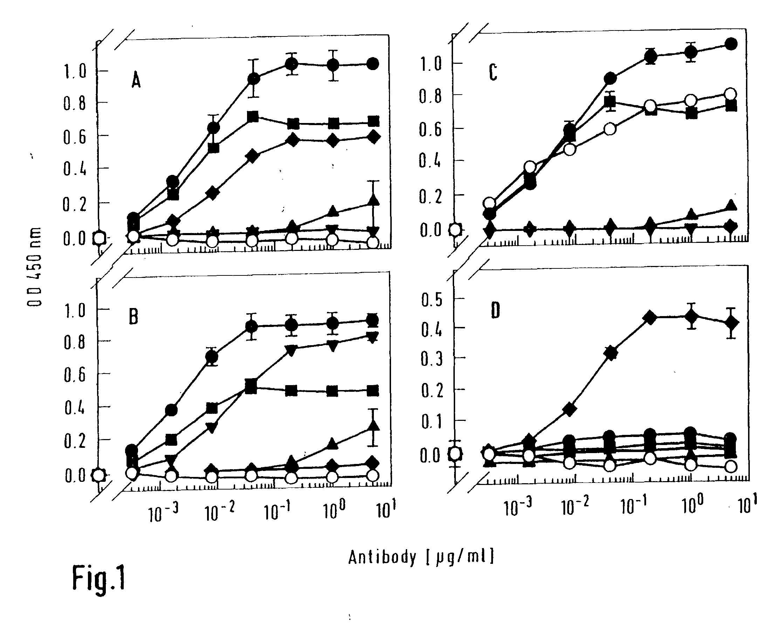

The monoclonal antibody 1409.F8 binds strongly to αvβ1, αvβ3, αvβ5 and αvβ6 integrins. In ELISA on purified human integrins, 14D9.F8 and 17E6 showed identical reaction profiles and similiar concentrations of antibodies were needed to obtain 50% maximal binding (Fig.1). 14D9.F8 did not react with αllbβ3, indicating that it was targetting the αv-chain, as it has been previously concluded from a FACS analysis (Mitjans, F. et al., J. Cell Sci. 108, 2825-2838 (1995)). 14D9.F8 had little effect on cell attachment mediated by αvβ5, and weak-to-moderate effects on the attachment mediated by αvβ3 and αvβ1 (Mitjans, F. et al., J. Cell Sci. 108, 2825-2838 (1995)). However, when it has been tested 14D9.F8 for its ability to affect cell attachment of HT-29 and UCLA-P3 carcinoma cells to fibronectin, it has been shown that it was extremely active. (Fig. 2)
14D9.F8 abolished HT-29 cell adhesion to fibronectin with an IC₅₀ of some 0.3 nM while on vitronectin up to 600 nM it was, rather, stimulatory. The reason for this is not clear. The binding of HT-29 to vitronectin was entirely inhibited by P1F6, and to fibronectin was insensitive to P1F6 but was abolished by 17E6. LM609 did not react with HT-29 or UCLA.P3 cells (Mitjans, F. et al., J. Cell Sci. 108, 2825-2838 (1995)) and had no effect on their attachment to vitronectin or fibronectin (Fig. 2A, B). Thus, an αv-integrin but not αvβ3 or αvβ5 modulates HT-29 adhesion to fibronectin, and this integrin is selectively blocked by Mab 14D9.F8, while the integrins mediating adhesion to vitronectin are inactivated by Mab P1F6 and this integrin is weakly inhibited by 14D9.F8.

To demonstrate that it was not a peculiarity of HT-29 cells, the inhibition experiments were repeated using UCLR-P3, a carcinoma cell from a different source, lung, which express high levels of αvβ5 and αvβ6 and obtained similiar results (Fig. 2 C,D).
Mab 14D9.F8 abolished cell adhesion to fibronectin, adhesion mediated mainly by 17E6-sensitive molecules-with some contribution of P4C10-sensitive molecules (presumably αvβ5 or αvβ1). On vitronectin 14D9.F8 inhibited up to 30% of cell attachment, but P1F6 abolished it. If, as believed, P1 F6 is selective for αvβ5 (Weinacker et al., J. Biol. Chem. 269, 6940-6948 (1994)), these data suggest that the integrin on UCLA-P3 mediating attachment to fibronectin is αvβ5, and this molecule is inhibited by 17E6 some 4 orders of magnitude strongly than by 14D9.F8. As in ELISA and FACS 17E6 and 14D9.F8 bind αvβ3, αvβ5 and αvβ6 with very similiar concentration dependency. This is an unexpected finding.
Melanoma cell lines uniformly express αvβ5, αvβ3 or αvβ1, but no αvβ6 or αvβ8 (Marshall, J.F. et al. Semin. Cancer Biol. 7, 129-138 (1996)). As shown in Fig. 2.E,F, on WM164 melanoma cells, attachment to fibronectin was insensitive to αv-inhibitors, while attachment to vitronectin was blocked by 17E6, and some 50% by 14D9.F8 and up to 80% by LM609. Addition of P1F6 to LM609 or 14D9.F8 completely inhibits WM164 attchment to vitronectin. This indicated this attachment was mediated by both LM609 and P1F6-sensitive molecules, and that the LM609, but not the P1F6, sensitive target was also inhibited by 14D9.F8.

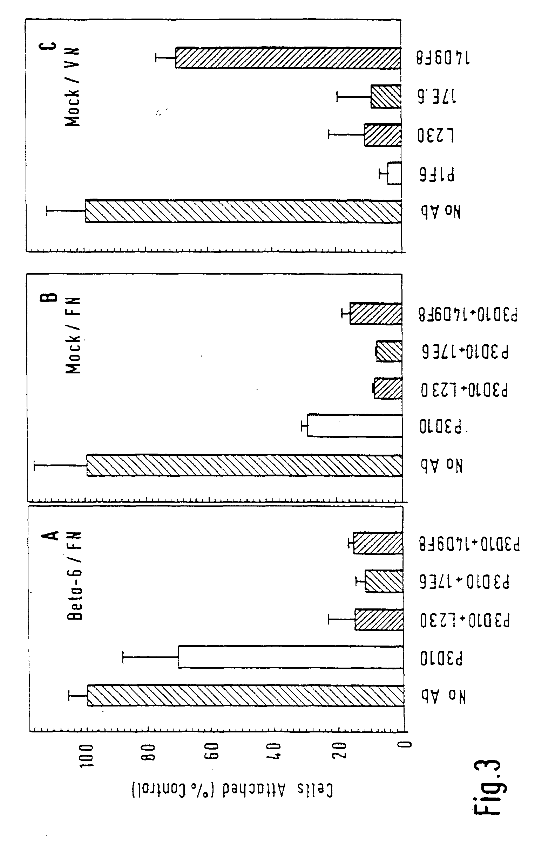

Many carcinoma cell express αvβ6 (Sheppard, D. (1996) Bioessays 18, 655-660). It was hypothesised that the carcinoma cell adhesion to fibronectin was mediated largely by αvβ6, and that 14D9.F8 was selectively inhibiting this molecule, while not inhibiting αvβ3 and αvβ5. To confirm this, it was measured the attachment of the SW480 cell line, transfected with full-length human β6, in the presence of 14D9.F8:
On fibronectin, the SW480-β6-transfectants were little affected by P3D10, while 14D9.F8, 17E6 and L230 were all potent inhibitors of attachment (Fig.3A). However, the attachment of the mock transfected SW480 on fibronectin, where they exclusively use α5β1 as adhesion receptor, could be strongly blocked using Mab P3D10 (Fig. 3B) (Weinacker, A. et al. J. Biol. Chem. 269, 6940-6948 (1994)) and 17E6, L230, and 14D9.F8 had only slight additional activity, possibly due to inhibition of αvβ1 (Marshall, J.F. et al. J. Cell Sci. 108, 1227-1238 (1995)).
Once again, on vitronectin, cell adhesion was inhibited strongly by P1F6 and 17E6, but was unaffected by 14D9.F8 (Fig. 3C). Together, these data show that 14D9.F8 selectively inhibits αvβ6 and αvβ3 more strongly than αvβ1, and does not affect αvβ5, although it ligates all of these integrins strongly.

The molecular details of this process are unknown, but a common interpretation of the data is that substantial alterations in integrin conformation occurs following ligand binding (Schwartz, M.A. et al. Ann. Rev. Cell Dev. Biol. 11, 549-599 (1995)). It is therefore favoured the possibility that when 14D9.F8 is bound to the αv-chain of αvβ6 and αvβ3 it prevents a motion of the β-chains against the αv-chain which is necessary to allow ligand binding. Sequence comparison shows potential sequences that show the necessary conservation between β3 and β6, and vary in β5 that could be involved (*unpublished observations*).

### EXAMPLES

### Example 1: Materials

*Animals* Mice for antibody production (female BALB/c; 8 weeks old) and for tumor models ("nude mice": female homozygotic athymic BALB/c nu/nu; 4-5 weeks old) were from Criffa (Barcelona, Spain). Nude mice were maintained in a sterile room in micro-isolator cages, and were given sterilized food and water *ad libitum.* All manipulations were performed in a laminar flow hood.
*Proteins:* Fibronectin (Ruoslahti et al., 1982), vitronectin (Yatohgo et al., 1988) were purified from fresh frozen human plasma.
Where not otherwise stated, all manipulations were at 20°C, and all washings were with calcium-magnesium free PBS ("PBS": 137mM NaCl, 2.8mM KCI, 8.1mM Na₂HPO₄, 1.5mM KH₂PO₄; pH7.4). PBS++ is PBS with added 1mM MgCl₂ and 1mM CaCl_{2.} Where not specifically stated, chemicals (Merck KGaA, Darmstadt) were of highest available purity. Cyclic peptides like RGDfV were synthesized according to known standard techniques (e.g. FEBS Let. 291, p. 50-54, 1991).
***Tumor cell lines and cultures**-* American Type Culture Collection (ATCC) supplied HT29 human carcinomas, the UCLA-P3 human lung adenocarcinoma (Cheresh et al., 1989) (Dr. D.A. Cheresh; Scripps), and WM164 melanoma (Dr. M. Herlyn, Wistar) (Herlyn et al., 1990). All cells were cultured at 37°C in 7.5% CO₂ 92.5% air in 90% RPMI 1640, 10% fetal calf serum (FCS) plus 2mM L-glutamine, and were consistently free of mycoplasma as evaluated by a proprietary test (Mycotect Kit; Gibco).

***Antibodies**-* Monoclonal antibody (MAb) fusions, ELISA screening, subcloning and maintenance of cultures were all performed using standard technologies (Harlow and Lane, 1988) unless otherwise specified.

### Example 2:

*Immunization* MAbs against the αvβ3 were produced by intraperitoneal (ip) injection of purified placental αvβ3 immobilized on Sepharose (80 µg αvβ3 on 80 µl Sepharose in 200 µl PBS) or of live M21 cells (1x10⁶ cells in 0.5ml PBS) every two weeks over twelve weeks. Four days after the last injection, PEG-induced fusion was performed using Friendly Myeloma (Ventrex) as partner. Antibodies to a 200kDa melanoma-associated surface protein were produced by immunising intact M21 cells (1x10⁶ cells in 0.5ml PBS).
*Screening* ELISA on receptors and on fixed M21 cells were used. For receptor ELISA, 96-well ELISA plates (Dynatech) were coated with purified αvβ3 (1µg/ml in PBS, 16h;4°C), blocked (1.5% skimmed milk in PBS; 1h;4 °C) and incubated with hybridoma supernatants. Bound immunoglobulins were detected with alkaline-phosphatase conjugate anti-mouse Ig (Dako) using p-nitrophenyl-phosphate as substrate. For cellular ELISA, UCLAP-3 cells on 96-well tissue culture plates were fixed (4% paraformaldehyde in PBS, 15 min. 20°C) and blocked (3% BSA, PBS; 1h;4 °C) before incubation with hybridoma supernatants and detection as in receptor ELISA. Positive hybrids were subcloned three times by limiting dilution and adapted to RPMI medium. Immunoglobulin isotype was determined using subclass-specific heavy-chain antibodies (Zymed) or light-chains antibodies (Promega).

Other murine MAbs used in the studies were the gift of our colleagues LM609 to α_{V}β₃, P4C10 to β1 integrin (Carter et al., 1990) (Telios) and AP3 to the β3 chain (Furihata et al., 1987) (ATCC).

### Example 3:

*Antibody Purification and Scaling Up-* For large scale purification, antibody supernatants were harvested from exponential phase cultures grown in roller bottles. The antibodies purified on protein A-Sepharose CL-4B (Pharmacia) were dialysed against PBS before sterile filtration (0.45 µm) and storage at -70 °C (Harlow and Lane, 1988). Purified antibodies were freed of endotoxins by passage over Kurimover-II columns (Kurita-Vater; Tokyo). This reduced the endotoxin levels from >250 IU endotoxin mg⁻¹ antibody to < 0.2 IU mg⁻¹ in the *Limulus* assay (Melvaer and Fystro, 1982). F(ab')₂ and F(ab)' fragments of 17E6 (murine IgG₁) were prepared by standard techniques of pepsin clevage and separation on protein-A columns (Pharmacia), followed by papain clevage and separation by gel filtration (Harlow and Lane, 1988).

### Integrin purifications

αvβ5 was purified from human placenta (Smith and Cheresh, 1988). Term placenta was minced and washed in ~2vols ice cold solution A (0.05% w/v digitonin, 2mM CaCl₂,2mM PMSF, pH7.4), then filtered. The retained material was extracted in ~4vols ice cold buffer B (100mM octyl-β-D-glucopyranoside [OG], 1mM CaCl₂, 2mM PMSF, in PBS) and centrifuged (12000gmax, 45min; 4 °C). The supernatant was re-circulated over a P3D10 column (16h;4 °C). After washing with buffer C (0.1 % NP-40 in PBS; ~10 cv) and buffer D (0.1 % NP-40, 2mM CaCl₂, 10mM Na-acetate; pH 4.5 ~10cv), bound material was eluted with buffer E (buffer D adjusted to pH 3.1). The eluant was neutralized with 3M Tris (pH 8.8), dialysed against buffer C, and concentrated ~10x using Aquacide II (Calbiochem). The purified receptors were stored at -70 °C.
Recombinant αᵥβ₃ and αᵥβ₆ were purified from baculovirus infected High five cells by affinity chromatography on LM609 (αᵥβ₃) and 17E6 (αᵥβ₆), with elution, concentration and storage as far αᵥβ₅.
α_{||b}β₃ was prepared from human platelets (Pytela et al., 1986). Outdated platelet concentrates were mixed with one volume of Tyrodes buffer, pelleted (1200gmax) and the pellet extracted (1h;20 ° C) with lysis buffer (50mM OG, 1mM MgCl₂, 1mM CaCl₂, 1µM MnCl₂, 2mM PMSF, 150mM NaCl, 50mM Tris-HCl; pH 7.4). After centrifugation (32000gmax , 30min; 4 °C) the supernatant was re-circulated (16h; 4 °C) over a GRGDSPK-conjugated CL-4B Sepharose column. The column was washed with lysis buffer (~10cv) and eluted with the GRGDSPK (3mg ml⁻¹ in 90% lysis buffer, 10% DMSO). The peak was concentrated ~5-fold , dialysed against modified lysis buffer (0.1% NP-40 substituted for OG) and stored at - 70°C. The integrin preparations were ∼ 95% pure as judged by anti-integrin ELISA using α-and β-chain specific monoclonal antibodies and by SDS-PAGE.

**Table 1**

| Antibody | Specificity | | Reference |
|---|---|---|---|
| | Binding | Inhibition | |
| 17E6 | αv | αv | (3) |
| 14D9.F8 | αv | αvβ6 - αvβ3 > αvβ1 | (3) |
| L230 | αv | αv | (5) |
| P3D10 | α5 | α5 | (2) |
| P4C10 | β1 | β1 | (6) |
| 4B7 | β1 | none | Marshal (personal commumcation) |
| AP3 | β3 | none | 1 (7) |
| 11D1 | β5 | none | this study |
| E7P6 | β6 | none | (1) |
| LM609 | αvβ3 | αvβ3 | (4) |
| P1F6 | αvβ5 | αvβ5 | (1) |

**Table 2**

| Cell | Integrin (Binds) | αvβ3 (VN,FN) | αvβ5 (VN) | αvβ6 (FN) | α5β1 (FN) | Reference |
|---|---|---|---|---|---|---|
| UCLA-P3 | | - | ― | ― | - | (1.3) |
| HT-29 | | - | ― | ― | - | (8.9) |
| WM-164 | | ― | - | - | - | This study |

## Claims

1. Use of the monoclonal antibody deriving from the hybridoma cell line, having the designation 271.14D9.F8, deposited under accession number DSM ACC2331, for selectively inhibiting *in vitro* the attachment of an αvβ6 integrin bearing cell to the integrin substrate fibronectin.

## Patentansprüche

1. Verwendung des monoklonalen Antikörpers, abgeleitet von der Hybridomzellinie mit der Bezeichnung 271.14D9.F8, hinterlegt unter der Hinterlegungsnummer DSM ACC2331, zur selektiven *in vitro*-Inhibierung der Bindung einer αvβ6-Integrin tragenden Zelle an das Integrin-Substrat Fibronectin.

## Revendications

1. Utilisation de l'anticorps monoclonal dérivé de la ligne cellulaire d'hybridome, ayant la désignation 271.14D9.F8, déposée sous le numéro d'accès DSM ACC2331, pour inhiber sélectivement *in vitro* l'attachement d'une cellule portant l'intégrine αvβ6 au substrat d'intégrine fibronéctine.
